# EUROPEAN PATENT APPLICATION

(11) **EP 3 673 728 A1**
(43) Date of publication of application: **01.07.2020**
(21) Application number: 18383001.7
(22) Date of filing: 28.12.2018
(51) Int. Cl.: A01G 33/00, C12M 1/00, C12M 1/12, C12M 1/26

(54) **A MICROALGAE-BASED SYSTEM FOR PRODUCING PRODUCTS AND A PROCESS MAKING USE THEREOF**

(71) Applicant: Global Biotech, S.L., 03560 El Campello Alicante (ES)
(72) Inventor: CHAPULI FERNÁNDEZ, Eloy, 03560 EL CAMPELLO (ALICANTE) (ES); ZAFRILLA REQUENA, Basilio, 03560 EL CAMPELLO (ALICANTE) (ES); COSTA, Henri George, 03560 EL CAMPELLO (ALICANTE) (ES); CATALA ARAÑO, Maria Carmen, 03560 EL CAMPELLO (ALICANTE) (ES); ZAFRILLA REQUENA, Guillermo, 03560 EL CAMPELLO (ALICANTE) (ES)
(74) Representative: Ponti & Partners, S.L.P

(57) **Abstract**

The present invention relates to a system for producing products employing microalgae. The present invention also relates to a process for producing products employing microalgae comprising the use of a system as disclosed in the present invention.

## Description

### FIELD OF THE INVENTION

The present invention relates to the field of technology applied to algae. In particular, the present invention relates to a system and a process for producing products employing microalgae.

### BACKGROUND

Microalgae are the building blocks of life and the most abundant primary producers of high added-value molecules from nature. Indeed scientists refer to them as the pharmacy of the world.

Several technologies are currently used to produce microalgae but most of them are too capital intensive to be implemented; the operative costs are too high to produce them at competitive prices and they can only be applied in some areas of the planet where there are specifics conditions to be implemented. In addition, the existing technologies for producing microalgae are not able to produce competitive products, compared to other natural sources, and this is because the premises of design were wrong and based on a different application (search of alternatives to biofuels). This is making difficult to access the market for most of the companies that are trying to switch their business and go into cosmetic and food/feed market. Indeed, Chlorella and Spirulina are the 2 types of microalgae that are competitive in the market at the moment, having a really big and profitable volume market, with a low CAPEX & OPEX, which are produced with a simple and cheap technology (Open Ponds). Apart from Open Ponds, other technologies (closed systems mainly) are capital intensive and it is difficult to currently make profitable plants.

Additionally, not all the microalgae can be grown in Open Ponds because this system is not suitable for species that are sensitive to the control of T, pH, CO₂ concentration, irradiation or sensitive to contamination. In these cases, closed systems, where all these variables can be managed in order to guarantee the productivity and the quality, are required. Furthermore, the market is demanding purer products with certain bioactivity and this means developing more sophisticated systems to produce such products and Open Ponds are not the optimal option.

The reactors which are currently used, either on an industrial or experimental scale, can be classified into two large groups:
A) Open Ponds
B) Closed reactors. Among the close reactors we have
   ∘ Horizontal
   ∘ Vertical
      ▪ Tubular
      ▪ Panels

### Open Ponds (see http://www.oilgae.com/algae/cult/op/op.html)

Open ponds can be categorized into natural waters (lakes, lagoons, ponds) and artificial ponds or containers. One of the major advantages of open ponds is that they are easier to construct (low CAPEX) and to operate than most closed systems. However, major limitations in open ponds include poor light utilization by the cells, evaporative losses, bad weather can stunt algae growth, diffusion of CO2 to the atmosphere, lack of control (T and pH) and requirement of large areas of land. Furthermore, contamination by predators and other fast growing heterotrophs have restricted the commercial production of algae in open culture systems to only those organisms that can grow under extreme conditions. Also, due to inefficient stirring mechanisms in open cultivation systems, their mass transfer rates are very poor resulting in a low biomass productivity.

The ponds in which the algae are cultivated are usually named as "raceway ponds". In these ponds, algae, water and nutrients circulate around a racetrack. With paddlewheels providing the flow, algae are kept suspended in the water and circulated back to the surface on a regular frequency. The ponds are usually kept shallow because the algae need to be exposed to sunlight and sunlight can only penetrate the pond water to a limited depth. Ponds are operated in a continuous manner, with CO2 and nutrients being constantly fed to the ponds, while algae-containing water is removed at the other end. One of the main problem of this technology is the high levels of contamination due to strains of bacteria or other phototrophic organisms often results in undesirable species taking over the desired algae growing in the pond, this fact reduce dramatically the homogeneity and reproducibility of the final product. The other big problem of open pound is the control of temperature .The high water volumes in which the algae grow should also be kept at a certain temperature in outdoor spaces. It usually becomes a difficult and expensive process. Another drawback is the uneven light intensity and distribution within the pond.

Summarizing, these open ponds are really simple, with low production costs and relative low operating costs, but at the same time it is not the most suitable system for the mass cultivation of most of microalgae species, being few strains capable of growing in such systems with an optimal yield.

### Closed reactors

### Horizontal Reactors

Horizontal closed photobioreactors, as described in WO2007025145A2, work according to a horizontal arrangement and are not as capital intensive as vertical reactors. Nonetheless, these horizontal reactors has some other limitations that makes them not suitable options for all the species:
- Difficulties to remove the O₂ generated by phototyntesis resulting in photoinhibition problems
- High OPEX
- Low productivity per m² and m³
- Only feasible in indoor conditions (outdoor conditions generate photoinhibition)

### Vertical Reactors

These reactors allow to overcome some of the main obstacles found in open system, such as the control of variables from the system (CO2, brightness, temperature ...), less contamination is produced and they require less land extensions to achieve the same production as in the open reactors, especially in case the layout is vertical (greater volume per unit area). (See PCT applications WO2007025145A2, WO2006 / 020177, WO 03/094598 and WO2007144441). However, all these systems procedures have the problem that their design is due to a massive approach to produce biofuels, and therefore they are capital intensive and their procedures are associated to high costs (cleaning, disinfection, evaporation of the culture water, energetically deficient (pumping, extraction,...)) and indirectly generate pollution. The higher efficiency that this kind of systems claim are thus not enough to be profitable in a competitive market.

### Simple bags

This system has as main (and only) advantages that it is very cheap and easy to implement. In contrast, the operation is not simple and does not allow to scale-up to commercial volumes. It is very difficult to control and therefore obtaininig quality products is not feasible.

### Flat Vertical Panel

Flat Panels are one of the best options to get good efficiencies. They are closed and simpler than closed previously mentioned photobioreactors. In addition, the optical path is normally smaller than in others (the photosynthetic yield is potentially higher than other systems). Nevertheless, there are some points to be solved to become a real industrial alternative:
- they are normally made of materials such as PMMA and PC and shaping these materials is very difficult;
- maintenance is required every now and then and this maintenece work is difficult due to the small gaps between panels;
- it is not clear how the process variables are controlled;
- shadow effect is present.

In view of above, the present inventors have developed a system that overcomes all these drawbacks and is simpler, cheap, and easy to manage, also being able to increase productivity, quality and the number of products to be produced.

### SUMMARY OF THE INVENTION

In a first aspect, the present invention relates to a system for producing products employing microalgae.

In a second aspect, the present invention also relates to a process for producing products employing microalgae comprising the use of a system according to the first aspect of the invention.

### BRIEF DESCRIPTION OF THE DRAWINGS

**Fig. 1**: Example of a closed productive unit, except for gas outputs, comprising the microalgae culture having each of said productive units a maximum height of 2 m
**Fig. 2**: Example of a grid for the productive unit. This is a structure for shaping and giving stiffness to the productive unit.
   Structure for shaping and giving stiffness to the productive unit
**Fig. 3A****-B:** Different views of a plurality of productive units (Module)

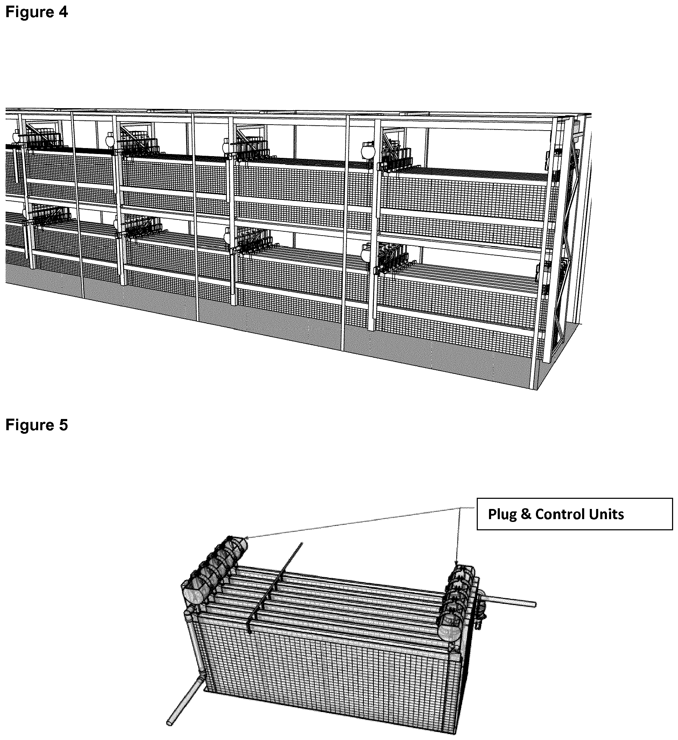

**Fig 4****.** Example of reactor. Combination of productive units in modules
**Fig.5****:** Example of a system control unit (also named herein as PLUG & CONTROL (PCU))
**Fig 6****.** Example of a cell (also named herein as PHOTOSTATE)
**Fig. 7A-B****.** Outdoor plant (A) where the configuration of the productive units changes to avoid shadow effect (B).
**Fig. 8****.** Example of other arrangements of the productive units. This proves that the flexible techecanonology that can be arranged as a mecano can be applied to the present invention.
**Fig. 9****:** Reactor of example 1.
**Fig. 10**: Reactor made up of 10 m long production units arranged in a linear way from example 2.
**Fig. 11A-B**: Different views of the plant for example 2.

### DETAILED DESCRIPTION OF THE INVENTION

In a first aspect, the present invention relates to a system for producing products employing microalgae, which comprises the steps of:
a) means for producing a microalgae culture, comprising said means:
   a1) a plurality of closed productive units, except for gas outputs, comprising the microalgae culture having each of said productive units a maximum height of 2 m;
   a2) means for generating turbulence of a gaseous fluid and for feeding thereof to the culture;
   a3) optionally, a structure for shaping and giving stiffness to the productive unit;
   a4) connective elements between the productive units;
b) a system control unit (also named as PLUG & CONTROL by the applicant) connected through narrow section capillaries to the top of the plurality of productive units and with a slope up to 10° thereover, comprising a plurality of probes and connective elements for emptying and filling by overflowing said productive units;
c) a cell (also named as PHOTOSTATE by the applicant) located out of said plurality of productive units provided with two photodiodes attached to the plurality of productive units controlling the oscillations of light inside the plurality units, wherein one of the photodiodes is oriented towards said productive units and the second photodiode is attached to said productive units, having said cell additionally a light emitter;
d) means of artificial and/or natural lighting;
e) means for transforming and/or extracting said product comprising at least one of the following:
   - means for mechanical separation;
   - means for washing with water;
   - means for drying;
   - means for breaking the microalgae;
   - means for purifying;
   - means for extracting with or without solvents.

In the context of the present invention, the term "product" or "products" obtained when using the system/process disclosed in the present invention is meant any commercial product resulting from microalgae, i.e. the origin of this commercial product is microalgae. Preferably, said product is a pharmaceutical product, a nutritional for animal or human consumption, a beverage product for animal or human consumption, a nutraceutical product, a cosmetic product, an aquiculture product, an agricultural product or a colorant.

This system of the present invention can be implemented in indoors or outdoor conditions.

Each of the elements comprising the system will be further disclosed with detail below:

### a) means for producing a microalgae culture

A vertical or horizontal arrangement of a plurality of productive units is called as "productive module". Preferably, the number of productive units is from 1 to 6. A group of productive modules would constitute a "reactor". In a preferred embodiment, said plurality of productive units in a1) are vertically parallel each other. This arrangement in productive modules is advantageous because of the possibility to diversify the production by modules and in case of incidence, for example due to contamination of the cultures, the modules can be isolated from each other, and thus the action on all the production is minimized.

In another preferred embodiment, the height of each of said productive unit without stacking is between 0.5 and 2 m, preferably between 0.5 and 1.5 m. When the productive units are stacked one over the other the height can be obviously higher than 2 m.

In another preferred embodiment, the distance between each productive unit is from 2 cm to 150 cm, preferably from 2 cm to 50 cm, more preferably from 2 cm to 20 cm. When the system is particularly in outdoor conditions, the distance between each productive unit is not usually lower tan 1 m.

Said plurality of closed productive units in a1) optionally further comprises at least one of:
- plastic pellets inside the productive units useful for reducing fouling; and
- enclosure for enclosing several productive units. This could be useful particularly in outdoor conditions since infrared sunlight could be used to raise the temperature of the culture in cold areas or cold seasons of the year (as a greenhouse) or filter it in those places that experience high temperatures or in warm seasons of the year. This would allow to reduce the temperature in the culture, also being able to cool the interior of the enclosure by means of evaporative cooling of the air via through water nebulizers and selective vents or with hot water pipes as a radiator. This enclosure would also provide a means of diffusing scattered light (minimizing the shadow effect, making the light reach each corner of the production unit). In addition, the enclosure would be integrated in the design of the productive unit, using it as a partition with a "canopy-type" mechanism to unfold the coverings of the enclosure. In case of indoor conditions, this enclosure would be designed so that it has the possibility of receiving artificial and natural light. This enclosure can be made of an opaque or transparent material (to use external light). It can also be flexible (greenhouse) or rigid. For indoor cultures the temperature control can be perfect by adding to the enclosure
air conditioning units or convention heat exchangers due to their low energy demand for their tempering.

As for the materials, said plurality of closed productive units are preferably made of a flexible plastic material selected from PE, PP, transparent PVC, PC, PMMA or a combination thereof, In another embodiment, said material is compatible with food/cosmetic/pharmacy industry when this is the destination of the product being produced according to the system of the present invention.

These productive units do not contain the air chamber used in the state of the art that found between the level of the culture and the upper limit of the productive unit. The production unit has exits (pipelines) to evacuate the gas. This minimizes the contact surface of the productive unit susceptible to staining due to the bubbling effect caused by the bubbling of CO₂ + air that is introduced through the base.

In another preferred embodiment, the gaseous fluid in a2) is selected from air, N₂, artificial CO₂, atmospheric CO₂, gases from industrial emissions or fermentation processes or a combination thereof. This gaseous fluid can be introduced by pulses or continuously. When using CO₂, the concentration would preferably be between 0,3 and 13% v/v, more preferably between 1 and 5% v/v.

The optional structure of a3) is optionally preferably made of metal, concrete or brick, more preferably said structure is made of metal and further comprises an intermediate layer made of a semi-rigid material protecting the plastic material. Optionally, this structure contains a reinforcement profile so as not to lose the flat shape. Nonetheless, although the addition of this structure is a preferred option, the productive unit may have enough strength and stiffness providing thereby the particular shape to the productive unit.

The connective elements between the productive units (a4) preferably comprises a fluid connective element(s) between two or more columns and a plurality of auxiliary elements for connecting fluids.

### b) a system control unit

In a preferred embodiment, the plurality of probes in the system control unit are at least a probe for measuring temperatures, a probe for measuring the culture concentration, a probe for measuring nutrient concentration, a probe for measuring pH and a probe for measuring CO₂ level; and, optionally, a probe for measuring suspended solids, a turbidimeter, a probe for measuring the cellular concentration, and a probe for measuring dissolved O₂. Obviously any other probe able to measure another parameter could be added to the system control unit.. If needed, a temperature control system may be added to said system control unit.

The productive units linked to this system control unit have a slight slope (1-10°) so that the system control unit is the highest point of the productive unit. In this way, it is ensured that all gasses to be removed (O2, air and non-absorbed CO2), in addition to foams and cellular debris, go upwards due to density difference favored by dragging gasses towards the system control unit. When getting to this unit they will be eliminated helping to avoid the fouling in the productive unit and prolonging its useful life and its productivity over time. It is likely that in the evacuation of air some liquid will be dragged in the form of foams, so that there may be a tank that will collect these draggings; in this way any spillage is always led to a collection point, thus avoiding any type of spill.

### c) a cell or cell system

Said cell located out of said plurality of productive units provided with two photodiodes attached to the plurality of productive units controls the oscillations of light inside the plurality units, i.e. the more light received, the higher the voltage and vice versa. It may also have an artificial light source to act in turbidity probe mode.

These voltage variations get directly or indirectly to the control system and generate a response. In this way, this cell constantly monitorizes and evaluates all the signals received from the sensors and integrates them into its control algorithm. In this way, the optimum culture of the production unit is achieved based on the amount of external light, photoperiods or meteorological broadcast (in the case of outdoor conditions, where this cell system is more advantageous, since it is constantly adapted to the external conditions, allowing the culture to always be in optimal conditions of photosynthetic performance). For the indoor conditions case, the cell is adapted to the provided lighting conditions, being able to adjust the latter with considerable reductions in energy consumption, especially during the starting process. It also has the advantages in both cases of monitoring online all the productive units, being able to obtain results of yields, trends, economic evaluations and other information useful for organizing the production.

In this way, the harvesting strategy can be adapted from more or less intermittent and intensive solutions to an almost continuous production of culture ready for processing. This also results in a substantial reduction in the dimensions of harvest, downstream and upstream equipment.

One of the key points is to maintain cell density at a value that allows full use of the perceived light so that all the cells have their photosynthetic machinery at 100% capacity, minimizing productivity losses due to cell respiration because of lack of light or excess photoxidation.

For indoor conditions the response of the cell system can modulate the lighting system, that is, if too much light intensity is detected inside the photo-bioreactor, it controls the system to reduce the intensity of the light source, in this case usually LEDs, thus helping to avoid stress by photoinhibition and unnecessary electrical consumption. Another option would be to harvest at the set point value, dilute the culture and allow the culture to recover the concentration before starting other harvesting cycle, i.e this work regime can be close to a continuous work regime if desired. We work with a set point and very little is harvested, so that in a few hours the set point is reached again and it is discharged again; in this way we can make several cycles a day (5 -10 cycles a day) and we approach to the desired continuous work regime that nobody has been able to develop up to now.

For both indoor and outdoor conditions, the measurement of this photosensor indicates when it is time to start harvesting by activating the medium addition pump. The medium enters through one side of each bag and the concentrated medium exits by overflow towards the waiting tank (towards the filter). The culture is progressively diluted so that light enters the reactor and a rise in the voltage emitted by the photosensor. When a threshold is reached, the pump stops and the cycle is restarted. According to the origin of the light detected by the photosensor, there are 3 working models:
1) The light source that receives the sensor (through the culture) is the environment of the system (either solar or LED).
2) The principle is the same as in 1), but it also computes values of total solar radiation (pyranometer). In this way it interferes (accepting, postponing or modulating) the decision made by the system focusing exclusively on the photosensor.
3) The light source is totally artificial, ignoring the environmental variations.

### d) means of artificial and/or natural lighting

In a preferred embodiment, said means of artificial and/or natural lighting are selected from:
- sunlight
- concentrator/distributor of sunlight, optionally through optical fiber,
- LEDs
- fluorescent tubes,
- light diffusers,
- distributors of artificial light, and
- a combination thereof.

These means of artificial and/or natural lighting can be arranged between productive units within a module, they can be external to the production modules or even submerged inside the productive unit. LEDs (artificial light or concentration of natural light) can be regulated according to the absorbance of the culture in each stage of growth. That is, a cell formed with photodiodes (see element c)) that calculates the absorbance at each moment and regulates the intensity of the LED illumination increasing gradually as the culture gains biomass to keep the linear illumination ratio with the cellular concentration, periods of photostress, harvest or what is required, is used. This system would gradually increase the intensity of the LEDs so that the photodiode always offered the same resistance to current flow. In this way, the lighting profile is kept constant or adjusted as required.

### e) means for transforming and/or extracting said product

In a preferred embodiment, the means for mechanical separation comprise at least one of:
- a submerged membrane filtration system (MBR);
- a tangential filtration system;
- means of flocculation and/or coagulation;
- means of centrifugation;
- means of filtration;
- means of electrocoagulation;
- an ultrasound system;
- means of mechanical vapor recompression (MVR);
- means of decantation.

In another preferred embodiment, the means for breaking the microalgae comprises at least one of:
- means for sonication;
- means for freezing;
- means for cavitation;
- means for mechanical disruption.
- means for enzymatic disruption;
- means for osmosis.

In another preferred embodiment, the means for drying comprises at least one of:
- means for thermally drying by hot air (spray drier);
- means of lyophilization (freeze-drying);
- multiple effect evaporator;
- fluidized bed dryer.

In another preferred embodiment, the means for purifying/extracting the product as disclosed herein comprises at least one of:
- Means for solvent extraction
- Means for water extraction
- Means for oil extraction
- Use of CO2 supercritical
- Use of CO2 supercritical cosolvent
- Use of Enzimatic extraction
- Rectification/destilation

Obviously, the different means for transforming and/or extracting the product would depend on the nature of said product. Once the microalgae culture has reached the proper concentration can be harvested. At this point and only by way of example:
- If the product of interest is biomass (for example Spirulina), the process steps for transformation and extraction are 1) filtration + 2) centrifugation + 3) drying (lyophilization)
- If the product of interest is an omega 3 acid, 1) filtration + 2) centrifugation + 3) extraction (for example with solvents)
- If the product of interest is a polysaccharide that excretes for example a species called *Porphyridium Cruentum* to the environment, 1) centrifugation to eliminate the biomass and use the aqueous phase that has the polysaccharide, 2) filtration to concentrate that polysaccharide, 3) wash and rinse to remove the salt and 4) if desired, dry or deliver it in liquid form.
- If the product of interest is the b-carotene of the *Dunaliella*, 1) filtration, 2) centrifugation + 3) break (freezing, cavitation, sonication) + 4) extraction with oil + 5) centrifugation again to eliminate the remaining biomass.

The present invention also relates to a process for producing a product employing microalgae comprising the use of a system according to the first aspect of the invention, said product preferably being a pharmaceutical product, a nutritional for animal or human consumption, a beverage product for animal or human consumption, a nutraceutical product, a cosmetic product, an aquiculture product, an agricultural product or a colorant.

The invention will now be further described by way of the following non-limiting examples.

### EXAMPLES

### Example 1

Facility: An indoor plant of 5000 m² of useful area, where 4000 m2 will be occupied by production reactors (formed by a set of productive units, grouped by modules in 3 levels); the other 1000 m² are for processing and stowage of the products, which in this example will have a final destination the cosmetic and food market.

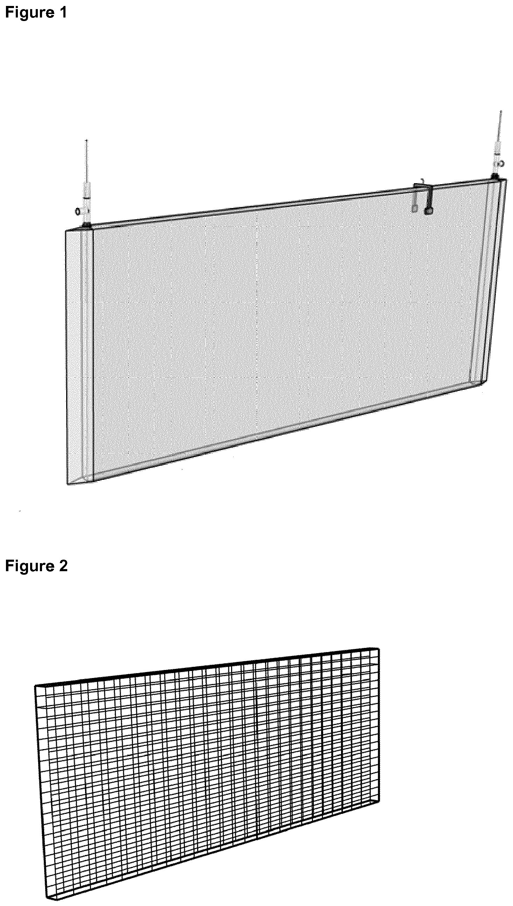

The production unit is made of flexible plastic (PE suitable for the food/cosmetic industry) and transparent, which is supported by a metal structure composed of grids whose dimensions (grid) are 100 x 50 mm (similar to figure 2). This grid provides rigidity to the plastic that contains the culture and also provides the dimensions that will define the efficiency of light uptake to the productive unit. Between the metallic structure and the flexible plastic a semi rigid plastic sheet made of PMMA is introduced for protecting the flexible plastic.
2.65 m long x 1 m high x 6 cm thick

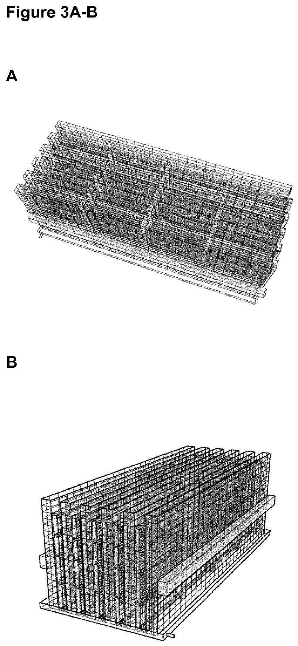

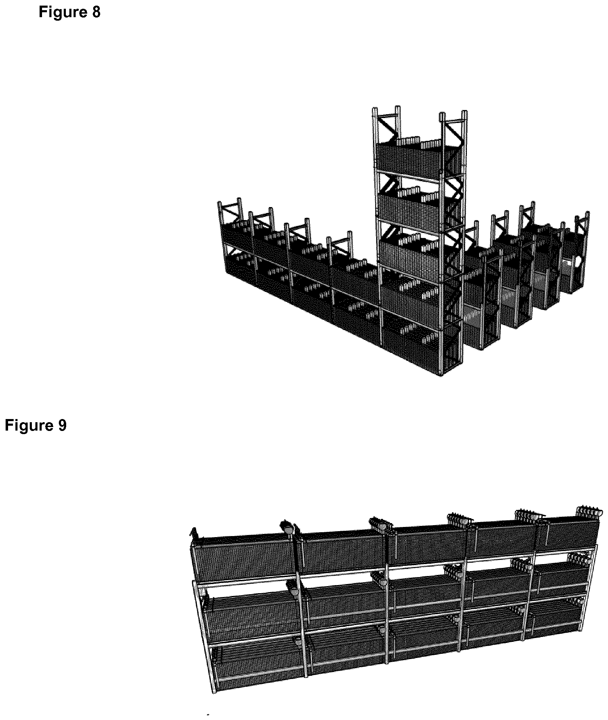

These production units are grouped in sets of 6 (in parallel) forming a productive module (see figure 3B), and as a mechanism they are assembled to form a reactor (see figure 9). In this example, the reactor has 3 module height and each height has 5 production modules.

Therefore, 1 reactor is constituted by 15 modules, and taking into account that a module in this example has 6 productive units (see figure 9), each reactor will have 90 production units, making a total of 960 modules (for 4000 m²), which represents a total of 5760 productive units for a total volume of 915 m³.

This volume extrapolated to 1 ha makes a ratio of 2 289 m³/ha, a value that is well above other systems mentioned in the state of the art; this value, being very interesting for an indoor system where the cost per m² can be important, would not represent an important value if it were not because the ratio of active surface exposed to light per unit volume is 33 m²/m³. This ratio is really high and well above the technologies mentioned in the state of the art, thus guaranteeing a photosynthetic yield above the average. In other traditional systems such as open pound where the depth of the pond can reach 30 cm this active surface value is around 3-5 m²/m³ of culture. In this way, the proposed system improves the productivity in one order of magnitude since it is well demonstrated that said ratio and productivity have a directly proportional relationship. Also, in this way the quantity of biomass obtained per unit of energy is enhanced and the costs associated with the processing of the culture are reduced since the volume to be treated per kg of obtained biomass obtained is also reduced by one order of magnitude. The CAPEX per unit volume is also greatly reduced, and all together makes the cost of production per kg of biomass much lower.

The working parameters used in this plant are the following (for the three products to be produced in the plant):
- Aeration 24 hours a day. 15 L/min per productive unit provided by a compressor at an average working pressure of 2.5 bar.
- Next to the air, food-grade synthetic CO₂ (Praxair) is introduced feeding the culture. A mixture is formed (air + CO2) in a mixing tank and the concentration to which the culture is fed is 1.5% in CO2.
- The productive unit is completely full thanks to the PCU.
- Each module, formed by 6 productive units, has one Plug & and Control Unit (PCU), which has the form of a closed expansion base and is connected to each of the productive units (PBC) through the top by means of narrow section capillaries, allowing to work with fully filled productive units; the PCU has exit holes to evacuate the gases (remaining air + CO2) and has 2 probes: a pH & T probe, and another one of dissolved O2. All the reactor modules have a PCU.

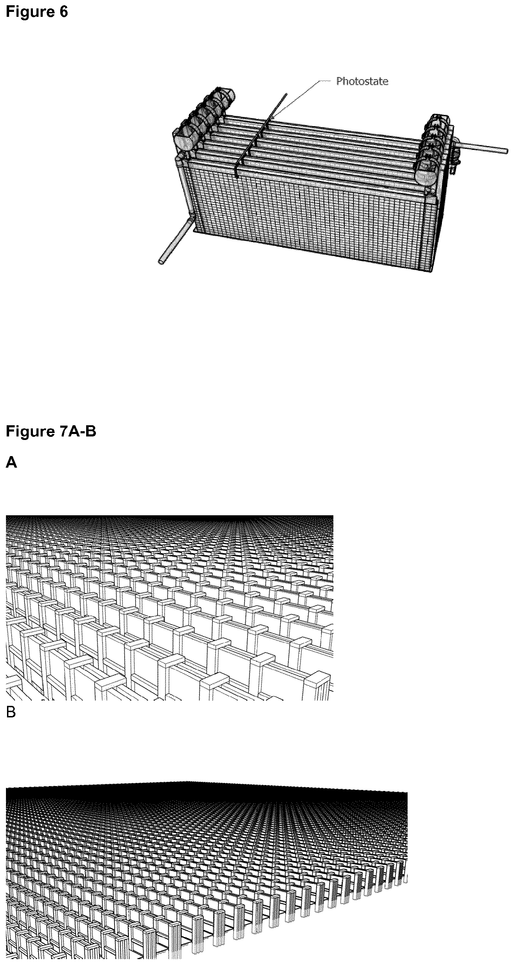

Another essential part of the system is the cell or cells (also named herein "Photostate". See figure 6). In this case, there will be 4 photostates per reactor; they will be placed in 4 productive units distributed in each reactor, and the control system will process the data taking as a value that determines the performance an average of the measurements taken both in time and for the photostates that are part of the reactor. The photostate is a measurement system that allows maintaining the optimal lighting and cell concentration conditions for a maximum efficiency in fixing the light provided to the reactor. It also allows a harvesting strategy very similar to a continuous reactor, greatly facilitating the design, construction and operation of downstream equipment after the reactor.

When the measurement of the photosensor identifies that the culture concentration is suitable to proceed with its harvesting, at that moment the water addition pump is activated by a side of the productive unit (from the permeate of previous cycles) consequently producing a simultaneous overflow through the PCU. The culture is progressively diluted so that the light enters into the reactor and a rise in the voltage is emitted by the photosensor. When a threshold is reached, the pump stops and the cycle is restarted.

To carry out this evaluation, the photostate performs 3 types of measurements using three elements: a light emitter and photosensor in the form of a turbidometric cell and a third externally oriented photosensor that evaluates the scattered ambient light available to the reactor.

The first type of measurement is using only the photosensor oriented inside the culture, in this way, the photostate evaluates the amount of real scattered light available to the culture.

The second type of measurement is using the light emitter and the opposed photosensor by measuring the cell concentration based on turbidity in a "traditional" manner.

The third type of measurement is using the photosensor oriented to the outside that determines the amount of scattered light available to the reactor to work.

Assessing these three signals and based on the mode of operation, outdoor or indoor, stress generation or simply photosynthetic efficiency, the control algorithm determines whether to harvest or not and to what extent to do so by the mechanism discussed above.

This operation allows the productive unit to work 5 months continuously. Once its shelf life is over, 100% of the module culture is harvested, and the productive unit is replaced. The productive unit is removed to take to recycling. A new productive unit is immediately placed and filled with inoculum prepared for such use. The activity of emptying, replacement of bag and filling is less than 3 hours per unit (the whole module is changed at the same time), so that the reduction in terms of non-operating time per productive unit is minimal with respect to its cycle of life.

It is harvested according to the intended concentration, which will depend on the species. In the following table, the intended concentration for each of the three species is shown:

**TABLE**

| | Spirulina | Tetraselmis | Chlorella |
|---|---|---|---|
| Intended concentration (g/l) | 1.20 | 0.90 | 1.00 |
| % extraction per cicle | 2.00% | 2.00% | 2.40% |
| Final concentration (g/l) after extraction | 1.176 | 0.882 | 0.976 |
| Extracted volume (l) per cycle and productive unit | 3.18 | 3.18 | 3.816 |

According to the reproduction rate of each of the 3 species, it is estimated that there will be 5 extraction cycles per day; each time the concentration of the intended concentration is reached (more or less every 4.8 h), a very small portion of the total volume of the productive unit is harvested by overflow (% extraction per cycle). This harvesting strategy responds to the importance of not altering the conditions of the culture at any time, and always having it in the exponential phase of growth (minimizing the adaptation phase). This is because the microalgae cultures do not generate biomass with the same efficiency whatever the concentration thereof is, there are concentrations where the amount of dividing cells and the amount of available light is optimal so that the efficiency of capture and therefore the biomass synthesis is maximum. The objective is to maintain the culture at this point constantly to maximize production and this is achieved by making small culture extractions that return the number of cells per ml within this optimum efficiency when the concentration of the culture rises too much.

As the harvested volume per cycle is reduced, this goes to an intermediate tank of small volume (10 times smaller than the standard size in this type of processes) before passing to the filter; in this way, the necessary equipment for processing is minimized (tanks, pumps, valves, pipes, etc.,). The permeate resulting from the filtration goes to another tank that collects this water, where it will be reconditioned to be reused again in the next growth cycle in the production unit, thus minimizing the daily water consumption of the plant. Through a treatment with ultraviolet light, a process with chlorine at low concentration and a final neutralization of this chlorine, the initial conditions of the culture medium are recovered allowing its use in the next cycle. It is important to mention that when working with such reduced work cycles, we not only minimize the necessary equipment, as mentioned above, but also minimize the residence times of these waters in the circuit; the smaller the volume is, the faster is the re-entry time of that volume in the circuit, minimizing the problems due to contamination and therefore maintenance of the reconditioned water.

Once the harvest has been carried out, the processing with the tangential filter starts by concentrating the culture approximately 50 times, reaching a concentration of approximately 75 g/l. The resulting product is a concentrate of microalgae, but it is still liquid.

This concentrate is led to a centrifuge reaching approximately 20% solids. This product is already a paste that has to be dried to give it the final format for marketing.

The pulp is dried in a lyophilizer to minimize degradation during the drying process, leaving the dried product with a residual humidity of 7% on average; in this way the bioactives that contain the biomasses are not degraded.

Based on this procedure, the mixed product mix in the plant consists of 3 products:
- Tetraselmis. It is a source of vitamins, antioxidants, omega 3, minerals and a wide variety of very interesting functional components both in food and in cosmetics.
- Spirulina, it has in its biomass metabolites whose biological activity provides health benefits. also for food and cosmetics
- Chlorella, like spirulina, it has metabolites of high biological activity.

The plant is located in the south of Spain, so it is necessary to take into account the working ranges of each of the species; tetraselmis is a species sensitive to high temperatures so the area of this species will have to undergo a rigorous temperature control that allows to work always below 27°C in summer.

The other two areas (Spirulina and Chlorella) will have a control system, but much less rigorous.

In all cases the temperature will always be above 20°C so that metabolically there is activity.

All the reactors of each of the 3 areas will be inside a receptacle (as a greenhouse) with plastic material to personally control each area. Each reactor will have a different set point.

For this example, the lighting will be artificial light using Led's as a source of light emission; in fact it will consist of a combination of white lights (daylight, 6500K) and red lights (3000 K), in a ratio of 2.5:1.

This ratio of lights is important because photosynthetic organisms do not absorb light throughout the visible spectrum but their pigment system is adapted to uptake light in the blue and red part of this spectrum.

To optimize the conversion of light into biomass, it is necessary to avoid losing energy by supplying light at wavelengths that these microorganisms cannot use (500-600 nm) and it is convenient to perform a mixed spectrum centered on the blue (450) and red (660) zone. This is the reason of why a combination of white leds (daylight) and pure red leds is ideal to maximize the uptake of energy by the phytoplankton.

### Example 2

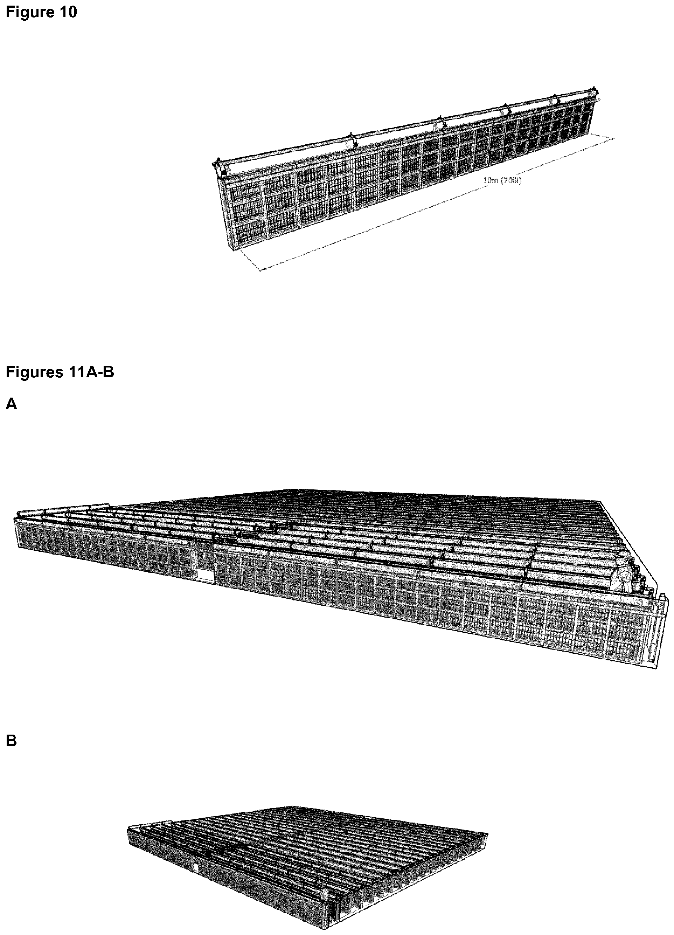

Facility: Outdoor production plant of 1 ha. in Alicante. On this occasion, each reactor is made up of 10 m long production units arranged in a linear way (figure 10), reaching a total length of 100 m. The lines between them are separated 0.75 m (figure 11A-B).

The plant, in addition to the ha. designated for production reactors, has an industrial warehouse of about 1000 m2 annexed to the land of 1000 m2 for processing and stockage of produced products; in this example the production will be for the aquaculture market.

The material containing the culture is flexible plastic (PE suitable for the food/cosmetic industry) and transparent, it is supported by a metallic structure (very similar to that shown in example 1). This, in order not to be deformed along the 100 m, is constituted by pieces of approximately 10 m that are welded together with respect to each other and of course there will be reinforcements to give rigidity to the final structure.

A grid gives rigidity to the plastic that contains the culture and gives it dimensions that will define the efficiency of light uptake to the productive unit
Reactor: 100 m long x 1.2 m high x 7 cm thick

These production units are separated from each other by 0.75 m distance, being arranged in parallel with one another. It is important to mention that the orientation of the productive units will be North-South to make the most of the sunlight, just as it is done with agricultural cultures.

This plant also has the peculiarity of being inside a 5-meter-high Polyethylene greenhouse, whose main function is to transform the direct light into diffuse light, increasing the photosynthetic efficiency of the culture (shadows of some reactors are avoided compared to others and the light is distributed homogeneously). In addition, this greenhouse system will keep the temperature stable between 22 and 27°C throughout the year, either by facilitating air currents inside the greenhouse and/or implementing an automated tempering system (heat pump, hot water exchangers, humidifying, etc). In addition, the greenhouse has a shading system that minimizes the effect of infrared radiation when required (thus preventing overheating of the culture in Summer).

According to this configuration, 123 lines of reactors of 100 m long each are arranged, which means a total volume of 885 m3.

The active surface ratio exposed to light per unit volume is 33.33 m²/m³. This ratio is really high and is still well above the technologies introduced in the state of the art, thus guaranteeing an photosynthetic yield over the average.

The working parameters used in this plant are the following (for the three products that the plant will produce):
- Aeration 12 hours a day. 12 L/min per productive unit providing a compressor at an average working pressure of 2.5 bar. During the night aeration is stopped because it is not necessary to feed with CO2 (there is no light, no photo-synthesis).
- Mixed with the air, CO2 is introduced from a source of emission of industrial gases from a cement plant located 400 meters from the plant; the emission gases are taken to a treatment unit that allows to extract the CO2 (with MEA solvent) and liquefy the CO2. This is the CO2 that is used to make a mixture (air + CO2) in a mixing tank and the concentration at which the culture is fed is 2% in CO2.
- The productive unit is completely full thanks to the PCU (see next point)
- Each linear reactor has a length of 100m (figure 1); this in turn consists of 10 units of 10 m long each, and each of them has a Plug & Control Unit (PCU), which has the form of a closed expansion base, and which is connected to each of the productive units (PBC) through the top by means of narrow section capillaries, allowing to work with the fully filled productive units; the PCU has exit holes to evacuate the gases (remaining air + CO2) and has a pH & T probe.

Another essential part of the system is the Photostate. The reactor (each line) has 4 Photostates distributed in the 100 m; the control system will process the data taking as a value that determines the action, an average of the 4 measures taken therein. Each reactor works independently of the rest of the reactors, so each reactor will have its average that defines the action of that particular reactor.

The Photostate is not only a system that captures information, but it processes it and acts according to the programming and instructions indicated by the person responsible for production.

The Photostate works in the following way:
∘ A photosensor measures the solar radiation that comes from the sun.
∘ A second photosensor is responsible for measuring the cell concentration of the culture at each moment.
∘ With the value of each of the previous photosensors, the Photostate evaluates the energy that is available and the concentration that the culture has. As a result of the integration of these two variables over time, the PHOTOSTATE determines if the system has reached its limit of luminous contribution in which the optimum of photosynthetic productivity is lost in a very robust manner. Thus, by integrating these two variables, the internal algorithm of the PHOTOSTATE calculates a partial dilution of the culture with fresh water up to a predetermined concentration value, facilitating in this way that the energy that reaches the culture is sufficient as to maintain optimal growth conditions.
∘ The system is also able to predict the rate of growth and the intended growth is not reached, an alarm will go off; the alarm sensitivity is determined by the plant manager.

As an example, we present a specific case that can be given during the month of June:
□ The horizontal daily irradiance of June 5 in Alicante is 6200 w/m2/day.
□ The system evaluates every hour if the concentration of the culture is above the value shown by the set point ked which as fixed by the producer.
□ Obviously the set point changes with the weather conditions; the set point is based on the work mode, which will depend on the energy that is arriving from the sun:
   ∘ Mode 1. Above 4500 W.h/m2/day
   ∘ Mode 2. Between 3500-4500 W.h/m2/day
   ∘ Mode 3. Below 3500 W.h/m2/day

Of course, the values assigned to each mode are manipulable by the person responsible for production.
□ For each mode, and each species, the set point changes (this is programmed by the person responsible for production and is modifiable):
   ∘ Mode 1. Spirulina: 1.2 g/L
   ∘ Mode 2. Spirulina: 1 g/L
   ∘ Mode 3. Spirulina: 0.8 g/L
□ The system harvests until the dry weight has been reduced by 0.1g/L (this is also a variable that the producer can change).
   ∘ Mode 1. Spirulina: 1.1 g/L
   ∘ Mode 2. Spirulina: 0.9 g/L
   ∘ Mode 3. Spirulina: 0.7 g/L
□ The system will make measurements every hour to show the evolution and quantity of light available during this specific day, of the culture depending on the working mode at each moment. When the culture reaches the set point again (depending on the mode, in this case 1.2 g/L), the culture is harvested until the set point previously marked drops to 1.1 g/L.
□ The system also contemplates the possibility of a day where clouds and clearings alternate. To indicate to the operator which mode to choose when the set point is reached to proceed with its harvest, the system considers all the intensities recorded during this specific day to establish an accumulated energy level. The system does not change the mode of harvest automatically, but an alarm goes off requiring rearm (by the person responsible) to validate the new mode. In this way, problems of changing modalities due to a momentary change in radiation are avoided. If we have the specific case in which the weather changes from a mode 1 to a mode 2 and the person responsible rearms and validates it, if the culture was at 1.1 g/L, it is quickly harvested until take it to 0.8 g/L (mode 2).
   If, on the other hand, we are in mode 2 and we pass to mode 1 because the weather is better, it is no longer harvested at 0.8 but at 1 g/L.
□ In this specific case (Spirulina, Mode 1, growth rate estimated i4n this mode 1 of 0.1 g/L/day), the starting concentration is recovered in 1 day. Therefore every day we have 1 extraction cycle of approximately 10% of the culture.

Harvesting is triggered, the fresh medium enters the reactor through the water addition pump on one side of the productive unit (from the previous cycle period), resulting in simultaneous overflow through the side opposite of the PCU. This concentrated effluent is directed towards the filter for processing. The culture is diluted progressively until the photovoltaic cell detects that the desired concentration has been reached. When a threshold is reached, the pump stops and the cycle is restarted.

This operation allows the productive unit to work continuously for 4 months. Once its shelf life is over, 100% of the module culture is harvested, and the productive unit is replaced. The productive unit is removed to take to recycling. A new productive unit is immediately placed and filled with inoculum prepared for such use. The activity of emptying, replacement of productive unit and filling is less than 24 hours per unit (a whole reactor is changed at a time), so that the reduction in terms of non-operational time per production unit is minimal with respect to its life cycle.

According to the reproduction rate of each of the 3 species, it is estimated that a cycle will be produced every 1-3 days depending on the season and the weather forecasts; each time the intended concentration is reached (24-72 h), a small percentage of the total volume of the productive unit is harvested by overflow (% extraction per cycle). This harvested strategy responds to the importance of not altering the conditions of the culture at any time, and always having it in the exponential growth phase (minimizing the adaptation phase).

As the harvested volume per cycle is reduced, this goes to an intermediate tank of small volume (10 times smaller than the standard size in this type of processes) before passing to the filter; in this way, the necessary equipment for processing is minimized (tanks, pumps, valves, pipes, etc.,). The permeate resulting from the filtration goes to another tank that collects this water, where it will be reconditioned to be reused again in the next growth cycle in the production unit, thus minimizing the daily water consumption of the plant. Through a treatment with ultraviolet light, a process with chlorine at low concentration and a final neutralization of this chlorine, the initial conditions of the culture medium are recovered allowing its use in the next cycle. It is important to mention that when working with such reduced work cycles, we not only minimize the necessary equipment, as mentioned above, but also minimize the residence times of these waters in the circuit; the smaller the volume is, the faster is the re-entry time of that volume in the circuit, minimizing the problems due to contamination and therefore maintenance of the reconditioned water.

Once the harvest has been carried out, part of the culture is processed and part is introduced into containers to distribute to nearby aquaculture companies that work with the green water technique. No drying or stabilization is required if the use is within 24 hours of being harvested. The culture to be processed is taken to a submerged membrane filtration system to reach a concentration of 70 g/L. The resulting product is a microalgae concentrate, but it is still liquid. This product can also be taken directly to the hatcheries to work with it in feeding artemia and fingerlings.

Another part of the culture is still concentrated in a centrifuge, reaching approximately 25% in solids. This product is already a paste that is being dried in a lyophilizer to minimize the degradation during the drying process, leaving the product dry with a residual humidity of 10% on average. In this way the bioactives that contain biomasses are not degraded and is suitable for use in aquaculture.

Based on this procedure, the mixed product produced in the plant consists of 3 products:
- Nannochloropsis. It is a source for Omega 3 (EPA) and other fatty acids, vitamins and antioxidants.
- Spirulina, it has in its biomass metabolites whose biological activity provides health benefits
- Isochrisis, source of omega 3 (DHA)

Since the plant is located in Alicante, it is necessary to take into account the working ranges of each of the species; Isochrisis and Nannochloropsis are species that must work always below 28°C, so the temperature (especially in summer) has to be rigorously controlled.

Spirulina will also have a control system but much less rigorous,
In all cases, the temperature will always be above 20°C so that photosynthetic efficiency is not metabolically lost.

All the reactors of each of the 3 areas will be inside a cabin (as a greenhouse) with plastic material to personally control each area. Each reactor will have a different set point.

### Example 3

Facility: 1 ha production plant in Mallorca. Each reactor is constituted by 2 levels (in different heights) one over the other. The second is identical and is placed on the top supported by the support structures required for such a function. In this way, the volume is twice (consequently, higher productivity per unit area, if it is able to ensure enough light energy to make grow the culture properly). Each level is formed by productive units of 10 m in length arranged in a linear way (similar to the figure in example 2), reaching a total length of 100 m each. The lines are separated 1 m between each other (similar to the figure in example 2). On this occasion, the reactor is not only illuminated with natural light but there is a contribution of artificial light as will be detailed in the description as follows.

The plant, in addition to the ha. designated for production reactors, has an industrial warehouse of about 1000 m2 annexed to the land of 1000 m2 for processing and stockage of produced products; in this example the production will be for extracting omega-3 product for feed.

The material containing the culture is flexible plastic (PE suitable for the food/cosmetic industry), transparent and strong enough to provide enough rigidity and strength to avoid using a metallic structure to provide the shape (figure 1) to the plastic, (very similar to that shown in example 1, but without the structure (the grid) providing the shape to the plastic.

In addition, the system is constituted by pieces of approximately 10 m that are welded together with respect to each other and of course there will be reinforcements to give rigidity to the final structure. In this example the reinformcements will be more robust that in example 2 because the the reactor is higher.

A grid gives rigidity to the plastic that contains the culture and gives it dimensions that will define the efficiency of light uptake to the productive unit
Reactor: 100 m long x 1.5 m high x 8 cm thick

These production units are separated from each other by 0.5 m distance, being arranged in parallel with one another. It is important to mention that the orientation of the productive units will be North-South to make the most of the sunlight, just as it is done with agricultural cultures.

On both sides of the reactor, LED strips are distributed linearly, according to the total power shown in the table below. There will be a combination of daylight and red light according to a ratio of 2.48. These lights are powered only by electricity from a photovoltaic plant attached to the production plant. The system will have batteries and will only use the energy of the batteries when the weather is such that the Photostate understands that the energy that reaches the culture is insufficient (night, cloudy, etc). Once the batteries are charged, the surplus will be used entirely in the lighting even if the day is totally sunny and the culture has enough energy; in this way we will provide an extra energy that will affect the production.

This plant has also the particularity of being inside a 8-meter-high polyethylene greenhouse, whose main function is to transform direct light into diffuse light, increasing the photosynthetic efficiency of the culture (shadows of some reactors are avoided compared to others and the light is distributed homogeneously); In addition, this greenhouse system will keep the temperature stable between 22 and 30°C throughout the year, either by facilitating air currents inside the greenhouse and/or implementing an automated tempering system (heat pump, hot water exchangers, humidifying, etc). In addition, the greenhouse has a shading system that minimizes the effect of infrared radiation when required (thus preventing overheating of the culture in summer).

According to this configuration, 188 lines of reactors of 100 m long each are arranged, distributed in two levels (different heights, one on top of the other), which supposes a total volume of 2256 m³.

As in the previous examples, the ratio of active surface exposed to light per unit volume is much higher than the rest of the systems from prior art.

The working parameters used in this plant are the following (for the three products to be produced in the plant):
- Aeration whenever there is light (artificial or natural). Since the number of hours to be illuminated is a variable, so will the number of hours of aeration; when there is no light, there is no aeration (there is no light, there is no photosynthesis, there is no introduction of air with CO2).
- Mixed with the air, CO2 is introduced from an industrial gas emission source from a Syngas production center (urban basins); the emission gases are considered clean enough and only the removal of combustible gases such as methane, propane, etc. is required. This is the CO2 to be used to make a mixture (air + CO2) in a mixing tank and the concentration at which the culture is fed is 2% in CO2.

- The productive unit is completely full thanks to the PCU (see next point)
- Each linear reactor has a length of 100m (figure 1); this in turn consists of 10 units of 10 m long each, and each of them has a Plug & Control Unit (PCU), which has the form of a closed expansion base, and which is connected to each of the productive units (PBC) through the top by means of narrow section capillaries, allowing to work with the fully filled productive units; the PCU has exit holes to evacuate the gases (remaining air + CO2) and has a pH & T probe.

Another essential part of the system is the Photostate. The reactor (each line) has 4 Photostates distributed in the 100 m; the control system will process the data taking as a value that determines the action, an average of the 4 measures taken therein. Each reactor works independently of the rest of the reactors, so each reactor will have its average that defines the action of that particular reactor.

The Photostate is not only a system that captures information, but it processes it and acts according to the programming and instructions indicated by the person responsible for production.

The Photostate works in the following way:
∘ A photosensor measures the solar radiation that comes from the sun.
∘ A second photosensor is responsible for measuring the cell concentration of the culture at each moment.
∘ A third sensor measures the radiation that really reaches the culture (between reactors), since it is necessary to remember that there are leds attached to both sides of the reactors.
∘ With the value of each of the previous photosensors, the Photostate evaluates the energy that is available and the concentration that the culture has. As a result of the integration of these two variables over time, the PHOTOSTATE determines if the system has reached its limit of luminous contribution in which the optimum of photosynthetic productivity is lost in a very robust manner. If there is no enough light, the system will give the signal so that the led's light up. If despite having enough light, the culture has simply reached the set point (concentration considered as maximum), the internal algorithm of the PHOTOSTATE calculates a partial dilution of the culture with fresh water up to a predetermined concentration value, facilitating in this way that the energy that reaches the culture is sufficient as to maintain optimal growth conditions.
∘ The system is also able to predict the rate of growth and the intended growth is not reached, an alarm will go off; the alarm sensitivity is determined by the plant manager. This allows to detect anomalous biological behaviors in the culture (contamination, or stress).

As an example, a specific case is presented so that it is clear what was commented before:
□ The system evaluates every hour if the concentration of the culture is above what the set point shows as established by the producer responsible.
□ Unlike the example 2, work modes are not used. According to the energy inputs, the growth rate of the culture that it will have and provides extra light with the LEDs to guarantee a minimum growth rate (0.08 g/L/day) is estimated.
□ When the energy that is between panels is below the level of what will allow to grow up to 0.1 g/L/day, the LEDs are turned on to reach the desired value. If it is already over it, only extra light will be provided with the led's in case the batteries are 100% charged.
□ The dilution will be 0.1 g/L, so that in 1 day the initial concentration will be recovered; in this way the extraction volumes are low and the culture is altered as little as possible.
□ Therefore, the system arrives at the set point (1 g/L). Harvesting is triggered, the fresh medium enters the reactor through the water addition pump on the side of the productive unit (from the permeate of previous cycles) resulting in simultaneous overflow through the opposite side of the PCU. This concentrated effluent is directed towards the filter for its processing. The culture is progressively diluted until the photovoltaic cell detects that the desired concentration has been reached (0.9 g/L in this particular case.) This value is modifiable.
□ The system will make measurements every hour to show the evolution of the culture and act as described above.
□ The system contemplates the possibility of passing a cloud in a timely manner, quickly entering artificial light, thus correcting the irradiance reduction.

Harvesting is triggered, the fresh medium enters the reactor through the water addition pump on one side of the productive unit (from the previous cycle permeate), resulting in simultaneous overflow through the side opposite of the PCU. This concentrated effluent is directed towards the filter for processing. The culture is diluted progressively until the photovoltaic cell detects that the desired concentration has been reached. When a threshold is reached, the pump stops and the cycle is restarted.

This operation allows the productive unit to work continuously for 8 months. Once its shelf life is over, 100% of the module culture is harvested, and the productive unit is replaced. The productive unit is removed to take to recycling. A new productive unit is immediately placed and filled with inoculum prepared for such use. The activity of emptying, replacement of productive unit and filling is less than 24 hours per unit (a whole reactor is changed at a time), so that the reduction in terms of non-operational time per production unit is minimal with respect to its life cycle.

According to the reproduction rate of each of the 3 species, it is estimated that a cycle will be produced every 1-3 days depending on the season and the weather forecasts; each time the intended concentration is reached (24-72 h), a small percentage of the total volume of the productive unit is harvested by overflow (% extraction per cycle). This harvested strategy responds to the importance of not altering the conditions of the culture at any time, and always having it in the exponential growth phase (minimizing the adaptation phase).

As the harvested volume per cycle is reduced, this goes to an intermediate tank of small volume (10 times smaller than the standard size in this type of processes) before passing to the filter; in this way, the necessary equipment for processing is minimized (tanks, pumps, valves, pipes, etc.,). The permeate resulting from the filtration goes to another tank that collects this water, where it will be reconditioned to be reused again in the next growth cycle in the production unit, thus minimizing the daily water consumption of the plant. Through a treatment with ultraviolet light, a process with chlorine at low concentration and a final neutralization of this chlorine, the initial conditions of the culture medium are recovered allowing its use in the next cycle. It is important to mention that when working with such reduced work cycles, we not only minimize the necessary equipment, as mentioned above, but also minimize the residence times of these waters in the circuit; the smaller the volume is, the faster is the re-entry time of that volume in the circuit, minimizing the problems due to contamination and therefore maintenance of the reconditioned water.

Once the culture has been harvested, this harvested culture is processed in order to extract the omega 3 contained in the biomass.

The culture to be processed is taken to a flocculation system consisting of a 10 m3 truncated cone-shaped tank that once filled is fed with an organic cationic flocculant that allows changing the load of the microalgae, favoring that they attach with each other and tend to decant. After the decantation, the concentrate is collected at the base of the tank (truncated cone-shaped).

The resulting biomass goes to the extraction stage. The extraction is carried out in a mixing tank with ethanol. The total micture biomass (with water) + ethanol reaches an alcoholic strength of 60%. It is mixed at 60°C for 1 hour and the resulting mixture is centrifuged. On one hand we will obtain an extract ethanol + water + lipid fraction. On the other hand, a moist biomass with a residual content of lipids is obtained.

The lipid fraction contains 40% in eicosopentanoic acid (EPA, omega 3) with respect to the rest of fatty acids, and that is exactly what we are looking for.

The mixture ethanol + water (residual) + lipid fraction is taken to a dryer that works under vacuum to avoid degrading the lipid fraction.

The fraction of ethanol + water goes to a distiller to purify the ethanol, which will be used again in a new cycle.

Based on this procedure, based on its content of omega 3 (EPA), only Nannochloropsis is produced, which is characterized by containing said omega 3.

Since the plant is located in Mallorca, it is necessary to take into account the working ranges for Nannochloropsis, as it is a species that must always work below 30°C, so the temperature (especially in summer) has to be controlled rigorously.

In all cases, the temperature will always be above 22°C so that photosynthetic efficiency is not metabolically lost.

## Claims

1. System for producing a product or products employing microalgae, wherein said system comprises:
a) means for producing a microalgae culture, comprising said means:
a1) a plurality of closed productive units, except for gas outputs, comprising the microalgae culture having each of said productive units a maximum height of 2 m;
a2) means for generating turbulence of a gaseous fluid and for feeding thereof to the culture;
a3) optionally, a structure for shaping and giving stiffness to the productive unit;
a4) connective elements between the productive units;
b) a system control unit connected through narrow section capillaries to the top of the plurality of productive units and with a slope up to 10° thereover, comprising a plurality of probes and connective elements for emptying and filling by overflowing said productive units;
c) a cell located out of said plurality of productive units provided with two photodiodes attached to the plurality of productive units controlling the oscillations of light inside the plurality units, wherein one of the photodiodes is oriented towards said productive units and the second photodiode is attached to said productive units, having said cell additionally a light emitter;
d) means of artificial and/or natural lighting;
e) means for transforming and/or extracting said product or products comprising at least one of the following:
- means for mechanical separation;
- means for washing with water;
- means for drying;
- means for breaking the microalgae;
- means for purifying;
- means for extracting with or without solvents.

2. System according to claim 1, wherein said plurality of productive units in a1) are vertically parallel each other.

3. System according to claim 1 or 2, wherein the height of each of said productive unit without stacking is between 0.5 and 2 m.

4. System, according to any of claims 1 to 3, wherein the distance between each productive unit is from 2 cm to 150 cm, preferably from 2 cm to 50 cm, more preferably from 2 cm to 20 cm.

5. System according to any of the preceding claims, wherein said productive units further comprises at least one of:
- plastic pellets inside the productive units;
- enclosure for enclosing several productive units.

6. System according to any of the preceding claims, wherein said productive units are made of a flexible plastic material selected from PE, PP, transparent PVC, PC, PMMA or a combination thereof.

7. System according to any of the preceding claims, wherein said optional structure is made of metal, concrete or brick.

8. System according to claim 7, wherein said optional structure is made of metal and further comprises an intermediate layer made of a semi-rigid material protecting the plastic material.

9. System according to any of the preceding claims, wherein said probes in b) are at least a probe for measuring temperatures, a probe for measuring the culture concentration, a probe for measuring nutrient concentration, a probe for measuring pH and a probe for measuring CO₂ level; and, optionally, a probe for measuring suspended solids, a turbidimeter, a probe for measuring the cellular concentration, and a probe for measuring dissolved

10. System according to any of the preceding claims, wherein the gaseous fluid in a2) is selected from air, N₂, artificial CO₂, atmospheric CO₂, gases from industrial emissions or fermentation processes or a combination thereof.

11. System according to any of the preceding claims, wherein said means of artificial and/or natural lighting are selected from:
- sunlight
- concentrator/distributor of sunlight, optionally through optical fiber,
- Leds
- fluorescent tubes,
- light diffusers,
- distributors of artificial light, and
- a combination thereof.

12. System according to any of preceding claims, wherein said means for mechanical separation comprise at least one of:
- a submerged membrane filtration system (MBR);
- a tangential filtration system;
- means of flocculation and/or coagulation;
- means of electrocoagulation;
- means of centrifugation;
- means of filtration;
- an ultrasound system;
- means of mechanical vapor recompression (MVR);
- means of decantation.

13. System according to any of preceding claims, wherein said means for breaking the microalgae comprises at least one of:
- means for sonication;
- means for freezing;
- means for cavitation;
- means for mechanical disruption;
- means for enzymatic disruption;
- means for osmosis.

14. System according to any of preceding claims, wherein said product or products obtained in step g) is a pharmaceutical product, a nutritional for animal or human consumption, a beverage product for animal or human consumption, a nutraceutical product, a cosmetic product, an aquiculture product, an agricultural product or a colorant.

15. A process for producing a product employing microalgae comprising the use of a system according to any of the preceding claims.
